# EUROPEAN PATENT APPLICATION

(11) **EP 0 586 154 A2**
(43) Date of publication of application: **09.03.1994**
(21) Application number: 93306618.5
(22) Date of filing: 20.08.1993
(51) Int. Cl.: C07K 15/06, A61K 7/42, A61K 37/02

(54) **Melanin formation-inhibitory protein, and its preparation and uses**

(30) Priority: 21.08.1992 JP 264025/92
(71) Applicant: KABUSHIKI KAISHA HAYASHIBARA SEIBUTSU KAGAKU KENKYUJO, Okayama-shi Okayama (JP)
(72) Inventor: Fukuda, Shigeharu, Okayama-shi, Okayama (JP); Suemoto, Yasuo, Okayama-shi, Okayama (JP); Kurimoto, Masashi, Okayama-shi Okayama (JP)
(74) Representative: Daniels, Jeffrey Nicholas

(57) **Abstract**

A novel melanin formation-inhibitory protein, which has a molecular weight of 90,000±20,000 and a pI of 5.5±0.5, exerts a tyrosinase formation-inhibitory activity in pigment cells but does not substantially inhibit the inherent tyrosinase activity. Thus the protein is advantageously used as a pharmaceutical and cosmetic in the prevention and/or treatment for local chromatosises such as chloasma, ephelis and sunburn, as well as systemic chromatosises such as addisonism.

## Description

The present invention relates to a protein, and its preparation and uses, more particularly, to a novel protein having a melanin formation-inhibitory activity derived from a human cell, and its preparation and uses.

Melanin is present in the skin and plays an important role in the protection of the body from the affects of ultraviolet rays. Melanin is also an important factor in medical science and cosmetology. It has been known that melanin is formed or synthesized in skin tissues. The presence of an excessive amount of melanin makes the skin dark, and the inhomogeneous distribution therein causes chloasma and ephelis, both of which are the defects in cosmetology. Conventionally, tyrosinase inhibitors such as vitamin C, glutathione and cysteine have been used to decrease the melanin level in the skin and to produce a pigmentation-lighted skin.

These tyrosinase inhibitors are, however, unfavorably stable and insufficient in their skin-whitening effect in viable cells. Hydroquinone and monobenzyl ether of hydroquinone (MBEH), which have been used as a tyrosinase inhibitor, exert a strong efficacy in realizing a pigmentation-lighted skin, but destroy the inherent physiological functions of the skin and cause side effects such as alphos, pigmentary disorder and contact dermatitis. In Pigment Cell Research, Vol.2, pp.123-125 (1989), K. L. Kreutzfeld et al. studied reasons why the ventral skin-tissues of frogs are whiter than the dorsal skin-tissues, and tried to extract a melanin formation-inhibitory substance from the ventral skin-tissues. As a result, they found and reported a protein having a molecular weight of about 300,000 and a melanin formation-inhibitory activity. The protein is, however, frog origin so that it has a limitation to be used to whiten human skins.

As described above, developments of a skin-whitening agent having a satisfiable safeness and efficacy has been in a great demand. The present invention is to provide a composition which has a satisfiable efficacy in the improvement of a dark skin and in the treatment of chloasma and ephelis, as well as having a satisfiable safeness and purity with a relatively-high specific activity, and to establish a preparation of a protein having a melanin formation-inhibitory activity derived from a human cell. The present invention also provides a novel technique useful in the study of the mechanism of melanin formation.

The present inventors have studied melanin formation-inhibitory substances produced by established human cell lines.

As a result, the present inventors purified and recovered a melanin formation-inhibitory protein from a culture supernatant of such an established human cell line by using sequential chromatographic techniques. The present inventors studied the physicochemical properties of the protein to find that it has a melanin formation-inhibitory activity, and established the preparation. Thus, the present inventors accomplished this invention. The present protein having a melanin formation-inhibitory activity has the following physicochemical properties:
(1) Molecular weight
   90,000±20,000 on sodium dodecyl sulfate polyacrylamide gel electrophoresis (SDS-PAGE);
(2) Isoelectric point
   pI=5.5±0.5;
(3) Ultraviolet absorption spectrum
   Exerting the maximum absorption spectrum at a wavelength of around 280nm;
(4) Solubility in solvent
   Soluble in water, physiological saline, phosphate buffer and Tris-Hcl buffer;
(5) Activity
   Exerting a melanin formation-inhibitory activity in pigment cells; and
(6) Stability of activity
   Inactivated in water (pH 7.4) at 80°C for 30 minutes;
   Stable in water (pH 7.4) at 4°C for one month.

The invention will now be further described by way of example only with reference to the accompanying drawings wherein:

FIG.1 shows an SDS-PAGE pattern of the present melanin formation-inhibitory protein.

FIG.2 shows a thermostability of the present melanin formation-inhibitory protein.

The preparation of the melanin formation- inhibitory protein according to the present invention is attained by allowing an inducer to act on a human cell such as leukocytes, lymphocytes and established cell lines capable of producing the protein. The established human cell lines satisfactorily usable in the invention are, for example, myelomonocytic cell lines such as HL-60 cells (ATCC CCL 240), U937 cells (ATCC CRL 1593) and HBL-38 cells as described in Japanese Journal of Cancer Research, Vol.79, pp.757-765 (1988); T-cells such as HPB-MLT (FERM BP-2430); and B-cells such as RAMOS cells (ATCC CRL 1596), all of which have a satisfactorily-high producibility of the present protein. The methods to proliferate such a human cell usable in the invention are in vitro and in vivo proliferations. The in vivo proliferation as described in Japanese Patent Publication No.54,158/81, wherein a cell needed to be proliferated is transplanted to a non-human warm-blooded animal, can be employed in the invention as an in vivo proliferation. By using the in vivo proliferation, a large amount of cells is readily prepared so that it is advantageously useful for such a purpose.

The following experiments will explain the present invention in detail.

### Experiment 1

### Preparation and physicochemical properties of protein having melanin formation-inhibitory activity

New born hamsters were injected with an antiserum, prepared from rabbits in conventional manner, to decrease their immunoreaction, subcutaneously transplanted with HPB-MLT cells (FERM BP-2430), and bred for 3 weeks in usual manner. Tumors subcutaneously formed in the hamsters were extracted, cut into pieces, dispersed and suspended in physiological saline. The resultant cells were washed with serum-free RPMI 1640 medium (pH 7.2), and resuspened in the same fresh medium to give a concentration of about 5x10⁶ cells/ml. The resultant cell suspension was added with one µg/ml of lipopolysaccharide of Escherichia coli, and incubated at 37° C for 48 hours to induce a melanin formation-inhibitory protein.

The resultant culture was subjected to centrifugation to obtain a supernatant which was then concentrated with an ultrafilter membrane having a molecular weight cut-off 6,000-10,000. The resultant filtrate was dialyzed against 20mM Tris-HCl buffer (pH 7.4) for 16 hours, and subjected to "DEAE-5PW column", a column product commercialized by Tosoh Corporation, Tokyo, Japan, to adsorb thereon a melanin formation-inhibitory protein. The column was washed with the same fresh buffer, and the melanin formation-inhibitory protein adsorbed on the column was eluted therefrom with a gradient buffer while increasing the concentration of saline from OM to 0.5M. The resultant active fractions were pooled and dialyzed against 25mM BisTris buffer (pH 7.1) for 16 hours, and the resultant solution containing an active protein was allowed to adsorb on "Mono P column" commercialized by Pharmacia LKB Biotechnology Uppsala, Sweden, and eluted from the column with a gradient buffer while decreasing the pH from 7 to 5, followed by recovering fractions containing the active protein. About 2mg of a purified product was recovered from 100L of the above-mentioned supernatant.

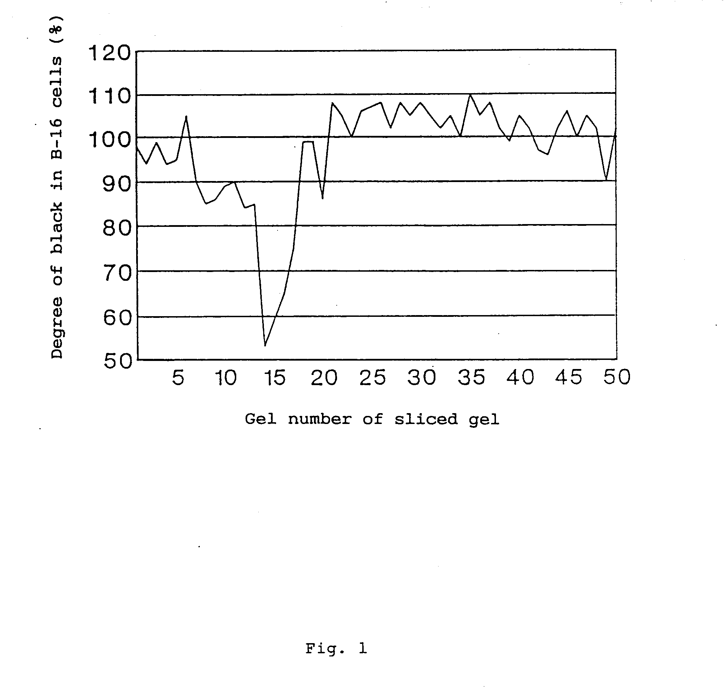

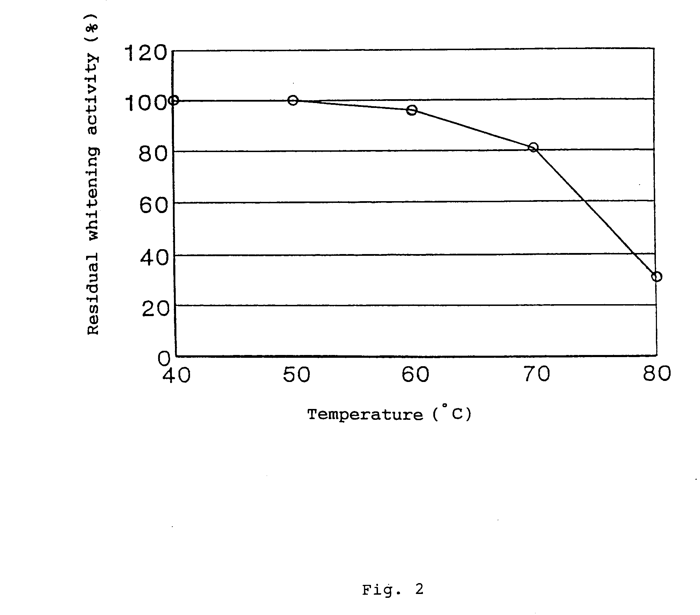

The physicochemical properties of the present melanin formation-inhibitory protein was studied with the purified product.
(1) Molecular weight
   In accordance with the method of Laemmli, Nature, Vol.227, pp.680-685 (1970), the purified product was subjected to SDS-PAGE. After completion of the electrophoresis, the resultant gel was sliced into pieces 2mm wide, which were then added in total with 250µl of Eagle's minimum essential medium supplemented with 10 v/v % fetal calf serum, and soaked therein at 4°C for 16 hours to extract a melanin formation-inhibitory protein. The activity of each extract from the sliced gels was determined with the following method. The results were as shown in FIG.1. In the figure, the numbers given in the axis of abscissa indicate the gel numbers of the sliced gels; and those in the axis of ordinate, the degrees of black of B-16 cells. FIG.1 shows that a melanin formation-inhibitory protein is present in fractions having a low degree of black. As evident from FIG.1, an active peak was observed in a part of gel containing a protein with a relatively-low mobility. The molecular weight of the melanin formation-inhibitory protein was 90,000±20,000 when determined in comparison with relative mobilities of marker proteins;
(2) Isoelectric point
   It was found that the purified product has a pI of 5.5±0.5 on chromatofocusing using "Mono P column";
(3) Ultraviolet absorption spectrum
   The purified protein exhibited the maximum absorption spectrum at a wavelength of around 280nm on "UV 250 spectrophotometry", a product of Shimadzu Corporation, Kyoto, Japan;
(4) Solubility in solvent
   Soluble in water, physiological saline, phosphate buffer and Tris-HCl buffer;
(5) Activity
   In accordance with the method in Cancer Research, Vol.42, pp.1994-2002 (1982), the melanin formation-inhibitory activity of the purified product was assayed: 4x10⁴ B-16 cells, a muse melanoma cell, were suspended in a 25cm² culture flask with 10ml of Eagle's minimum essential medium supplemented with 10 v/v % fetal calf serum, and cultured at 37°C under 5 v/v % CO₂ conditions. The cultivation was carried out for 5 days while replacing the medium with the same fresh one but supplemented with a prescribed amount of the purified product at days 0 and 3 after the initiation of the culture. After completion of the culture, the resultant cells grown on the inner walls of the culture flask were washed with phosphate buffer (pH 7.2) supplemented with 0.8 w/v % salt, added with trypsin and ethylenediamine tetraacetic acid (EDTA) to detach cells from the walls, and recovered by filtration. The cells recovered on a filter paper were dried and subjected to a densitometer in order to determine the reflection absorption (degree of black) at a wavelength of 500nm. Based on the above-mentioned method, one unit of melanin formation-inhibitory activity was defined as an activity which was observed when an absorbance in a test group was lowered to 1/2 of that of control group. By using a solution containing 2µg/ml of the purified protein, the melanin formation-inhibitory activity was determined with the above-mentioned method. As a result, the solution had 30 units/ml of melanin inhibitory activity.
(6) Stability of activity
   The purified protein was heated in a solution (pH 7.4) at a temperature in the rage of 40-80°C for 30 minutes, and assayed its activity with the above-mentioned method. The result was as shown in FIG.2. As evident from FIG.2, the purified product is inactivated at a temperature of 80°C or higher.
   While no substantial loss of activity was observed in the purified product after the storage at pH 7.4 and 4°C for one month.

### Experiment 2

### Functional mechanism of melanin formation-inhibitory protein

Intact B-16 cells as a control and those, which had been whitened by the treatment with the present melanin formation-inhibitory protein, were subjected to the following analyses:
(1) Quantitative analysis of melanin
   According to the method of Ito et al., Analytical Biochemistry, Vol.144, pp.527-536 (1985), the eumelanin and pheomelanin in B-16 cells were quantitated. As a result, as shown in Table 1 the level of eumelanin in B-16 cells, which had been treated with the melanin formation-inhibitory protein, was 1/15 of that of control. It means that the degree of black in B-16 cells was lowered to 1/15. While no significant change in the level of pheomelanin in B-16 cells was observed. In Table 1, the values in the columns of melanin content indicate a melanin content (µg) per 1x10⁷ B-16 cells.
(2) Assay of tyrosinase activity
   Cells which had been treated with or without the purified protein were busted by suspending them in 5-fold volumes of 0.25M sucrose solution, and subjecting the resultant suspension to a repeated freezing and thawing. The resultant mixture was centrifugally separated into fractions of extract and sediment, followed by assaying the activity of each fraction in accordance with the method of Hamada et al., British Journal of Dermatology, Vol.86, pp.385-394 (1972). The enzymatic activity was expressed based on the definition of that one unit (U) is an activity which decomposes one µmole of substrate per minute. As a result, as shown in Table 1 B-16 cells treated with the purified protein substantially lost their tyrosinase activity (Table 1). The values of tyrosinase activity in the column of tyrosinase activity in Table 1 indicate a tyrosinase activity per 1x10⁷ B-16 cells (mU).
   No change was observed when the melanin formation-inhibitory protein was added to the tyrosinase assay system, and this means that the present protein does not directly inhibit the tyrosinase activity.
(3) Determination of expression percentage of tyrosinase gene (mRNA)
   The method used in the following experiment is a conventional technique as described by M. Muramatsu, Labomannual Genetic Engineering, Published by Maruzen Co., Ltd., Tokyo, Japan (1988).
   RNAs of B-16 cells were prepared in usual manner from those treated with or without the purified protein, and subjected to an agarose electrophoresis. Thereafter, the separated RNAs were transferred onto a cellulose membrane by the blotting technique. The resultant RNAs on the cellulose membrane were subjected to the northern hybridization by using as a probe a ³²P-labelled cDNA of mouse tyrosinase as described in The ENBO Journal, Vol.7, pp.2,723-2,730 (1988). The resultant cellulose membrane was closely attached to an x-ray film, and the resultant was subjected to a radioautography. The expression percentage of the tyrosinase gene was evaluated based on the shade of colors of RNA bands hybridized with the probe. As a result, as shown in Table 1 no substantial difference in the expression percentage of tyrosinase gene was observed both in the cells treated with and without the purified protein. In Table 1, the values in the column of expression percentage of tyrosinase gene (mRNA) are expressed by relative values.

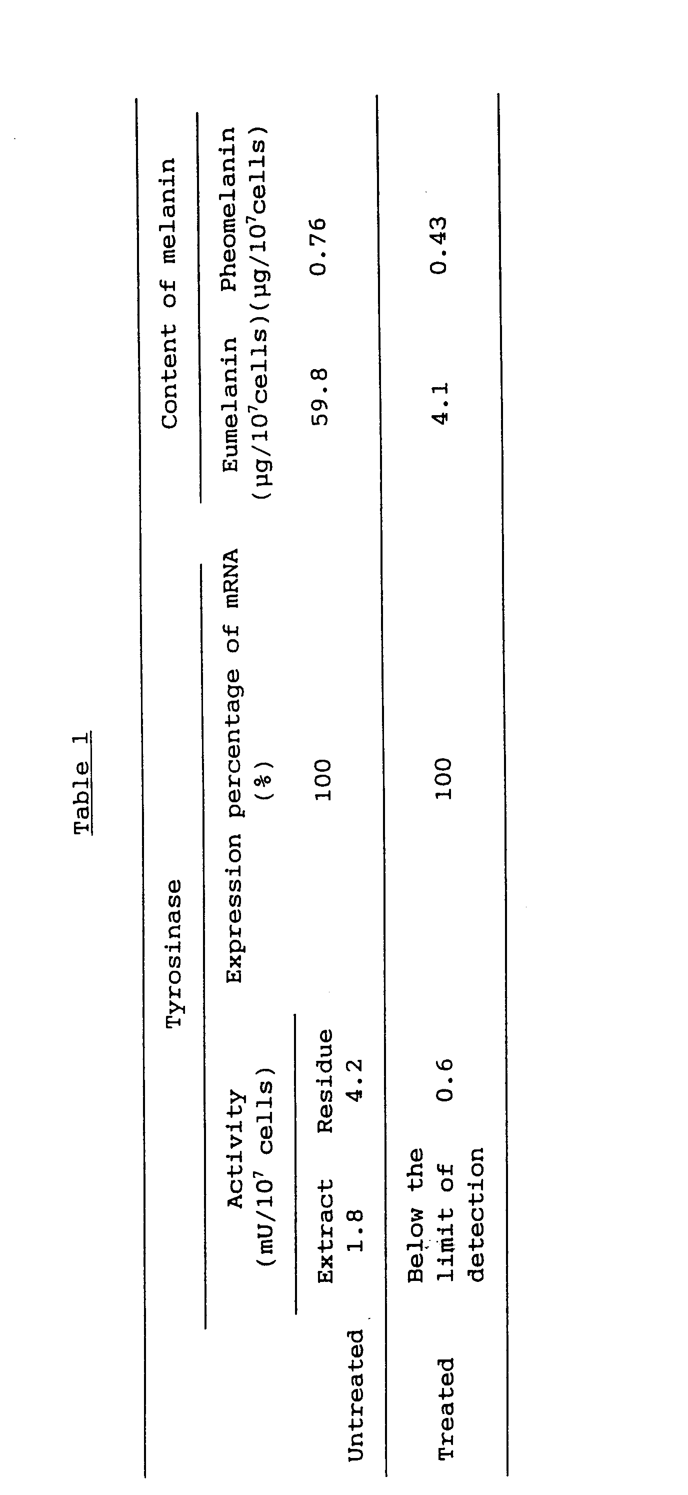

Based on these results, it is concluded that the present melanin formation-inhibitory protein does not directly inhibit the melanin formation or tyrosinase activity, but inhibits a process after the translation of a gene coding tyrosinase into a protein.

### Experiment 3

### Acute toxicity test

Acute toxicity of the melanin formation-inhibitory protein obtained in Experiment 1 was tested with 20-day old mice. As a result, it was revealed that the LD₅₀ of the protein is 150,000 units/kg or higher when orally and intraperitoneally administered to mice, as well as being administered to mice by the application to their grained skins. As evident from the above experiment, the present melanin formation-inhibitory protein has a strong melanin formation-inhibitory activity, i.e a satisfactorily-high skin-whitening effect, as well as having a satisfactorily-high safeness in view of its effective dose.

Skin-whitening agents containing the present melanin formation-inhibitory protein can be administered at a dose of 0.01-10,000 units/day/adult, based on the amount of the protein, on a dry solid basis (d.s.b.); preferably, 0.01-1,000 units/day/adult in systematic administrations such as intramuscular injections and the like; and 0.01-10,000 units/day/adult in oral administrations such as internal medicines, and percutaneous- and permucosal-administrations such as milky lotions and creams. The dose is suitably changed dependently on the administration route and/or patient's symptom. In order to prevent and/or treat local chromatosises such as chloasma, ephelis and sunburn, as well as systematic chromatosises such as addisonism, the melanin formation-inhibitory protein can be used alone and suitably used as a skin-whitening agent in combination with biologically active substances, nutrient agents, bases, fillers, excipients and the like, to meet to their final formulation such as pharmaceuticals and cosmetics, as well as to their actual use.

The preferred preparations of the present melanin formation-inhibitory protein will be described in Examples A.

### Example A-1

### Melanin formation-inhibitory protein

### Purification of melanin formation-inhibitory protein from HL-60 cells (ATCC CCL 240)

A seed culture of HL-60 cells (ATCC CCL 240) was allowed to proliferate in a culture flask in conventional manner, and similarly as in Experiment 1 the proliferated cells were added with an inducer to form a melanin formation-inhibitory protein which was then purified and recovered. About 100µg of a purified specimen was obtained from 10L of the culture supernatant. The melanin formation-inhibitory protein thus obtained has the same physicochemical properties as the protein in Experiment 1.

The product can be used as a skin-whitening agent in pharmaceuticals such as injections, orally administrable agents, externally administrable agents, and bath salts, as well as in cosmetics such as milky lotions, packs and creams; and exerts a relatively-high effect in the prevention and/or treatment for local chromatosises such as chloasma, ephelis and sunburn, as well as systemic chromatosises such as addisonism.

### Example A-2

### Melanin formation-inhibitory protein

A seed culture of RAMOS cells (ATCC CRL 1596) was allowed to proliferate in hamsters similarly as in Experiment 1, and the proliferated cells were added with an inducer to form a melanin formation-inhibitory protein similarly as in Experiment 1, followed by purifying and recovering the resultant protein. About one mg of a purified protein was obtained from 50L of the culture supernatant. The melanin formation-inhibitory protein thus obtained has the same physicochemical properties as the melanin formation-inhibitory protein in Experiment 1.

Similarly as the product in Example A-1, the product can be used as pharmaceuticals and cosmetics, and exerts a satisfactorily-high effect in the prevention and/or treatment for chromatosises.

The following Examples B will illustrate skin-whitening agents containing the present melanin formation-inhibitory protein as an effective ingredient.

### Example B-1

### Injection

One thousand units of a melanin formation-inhibitory protein, obtained by the method in Experiment 1, was dissolved in 100ml of a physiological saline containing 1 w/v % human serum albumin, and the resultant mixture was membrane filtered in usual manner. Two ml aliquots of the resultant filtrate were distributed to vials, freeze dried, and cap-sealed to obtain a lyophilized injection. The product is used by dissolving it in a sterilized water for injection, prior to use.

The product can be advantageously used as a skin-whitening agent in the prevention and/or treatment for local chromatosises such as chloasma, ephelis and sunburn, as well as systemic chromatosises such as addisonism.

### Example B-2

### Cosmetic (milky lotion)

To 100ml of a base in the form of a milky lotion, prepared in usual manner, 1,000 units of a melanin formation-inhibitory protein prepared by the method in Experiment 1, and the resultant mixture was subjected to a homogenizer to obtain a milky lotion. The product can be advantageously used as a skin-whitening agent in the prevention and/or treatment for chromatosises such as chloasma, ephelis and sunburn.

As described above, the present invention is to establish a preparation of a novel melanin formation-inhibitory protein which exerts a melanin formation-inhibitory activity without substantially inhibiting the enzymatic activity of tyrosinase, but inhibiting the synthesis of tyrosinase in pigment cells, and to establish a skin-whitening agent containing the protein as an effective ingredient.

The present melanin formation-inhibitory protein exerts a relatively-strong melanin formation-inhibitory activity and skin-whitening effect so that it exerts a relatively-high skin-whitening effect when used as a skin-whitening agent in the prevention and/or treatment for local chromatosises such as chloasma, ephelis and sunburn in pharmaceuticals such as injections, orally administrable agents, externally administrable agents and bath salts, as well as in cosmetics such as milky lotions, packs and creams.

The present melanin formation-inhibitory protein has a satisfactorily-high safeness and is used without side effects in view of its effective dose, and this renders it industrially useful in the fields of pharmaceuticals and cosmetics.

While there has been described what is at present considered to be the preferred embodiments of the invention, it will be understood the various modifications may be made therein, and it is intended to cover in the appended claims all such modifications as fall within the true spirits and scope of the invention.

## Claims

1. A protein which has the following physicochemical properties:
(1) Molecular weight
90,000±20,000 on sodium dodecyl sulfate polyacrylamide gel electrophoresis (SDS-PAGE);
(2) Isoelectric point
pI=5.5±0.5;
(3) Ultraviolet absorption spectrum
Exhibiting the maximum absorption spectrum at a wavelength of around 280nm;
(4) Solubility in solvent
Soluble in water, physiological saline, phosphate buffer and Tris-HCl buffer;
(5) Activity
Exerting a melanin formation-inhibitory activity in pigment cells; and
(6) Stability of activity
Inactivated in water (pH 7.4) at 80°C for 30 minutes.
Stable in water (pH 7.4) at 4°C for one month.

2. A protein according to claim 1, which exerts a tyrosinase formation-inhibitory activity in pigment cells.

3. A protein according to claim 1 or 2 which is derived from a human cell.

4. A protein according to claim 3, wherein said human cell is a member selected from the group consisting of HL-60 cell (ATCC CCL 240), U937 cell (ATCC CRL 1593), HPB-MLT cell (FERM BP-2430) and RAMOS cell (ATCC CCL 1596).

5. A process for preparing a protein, which comprises:
allowing an established human cell line capable of producing a protein to cultivate in a nutrient culture medium to form said protein, and
recovering said protein from the resultant culture; said protein having the following physicochemical properties:
(1) Molecular weight
90,000±20,000 on sodium dodecyl sulfate polyacrylamide gel electrophoresis (SDS-PAGE);
(2) Isoelectric point
pI=5.5±0.5;
(3) Ultraviolet absorption spectrum
Exhibiting the maximum absorption spectrum at a wavelength of around 280nm;
(4) Solubility in solvent
Soluble in water, physiological saline, phosphate buffer and Tris-HCl buffer;
(5) Activity
Exerting a melanin formation-inhibitory activity in pigment cells; and
(6) Stability of activity
Inactivated in water (pH 7.4) at 80°C for 30 minutes.
Stable in water (pH 7.4) at 4°C for one month.

6. A process according to claim 5, wherein said protein exerts a tyrosinase formation-inhibitory activity in pigment cells.

7. A process according to claim 5 or 6, wherein said human cell line is a member selected from the group consisting of HL-60 cell (ATCC CCL 240), U937 cell (ATCC CRL 1593), HPB-MLT cell (FERM BP-2430) and RAMOS cell (ATCC CCL 1596).

8. A composition, which comprises an effective amount of a protein as an effective ingredient, said protein having the following physicochemical properties:
(1) Molecular weight
90,000±20,000 on sodium dodecyl sulfate polyacrylamide gel electrophoresis (SDS-PAGE);
(2) Isoelectric point
pI=5.5±0.5;
(3) Ultraviolet absorption spectrum
Exhibiting the maximum absorption spectrum at a- wavelength of around 280nm;
(4) Solubility in solvent
Soluble in water, physiological saline, phosphate buffer and Tris-HCl buffer;
(5) Activity
Exerting a melanin formation-inhibitory activity in pigment cells; and
(6) Stability of activity
Inactivated in water (pH 7.4) at 80°C for 30 minutes;
Stable in water (pH 7.4) at 4°C for one month.

9. A composition according to claim 8, which is a skin-whitening agent.

10. A composition according to claim 8 or 9, which exerts a tyrosinase formation-inhibitory activity in pigment cells.

11. A composition according to any one of claims 8, 9 or 10, which is in the form of a cosmetic or a pharmaceutical.

12. A composition according to any one of claims 8 to 11, the dose of which is 0.01-10,000 units /day/adult of said protein.

13. A composition according to any one of claims 8 to 12, wherein said protein is prepared by a process comprising:
allowing an established human cell line capable of producing a protein to cultivate in a nutrient culture medium to form said protein, and
recovering said protein from the resultant culture.

14. A composition according to claim 13, wherein said human cell line is a member selected from the group consisting of HL-60 cell (ATCC CCL 240), U937 cell (ATCC CRL 1593), HPB-MLT cell (FERM BP-2430) and RAMOS cell (ATCC CCL 1596).
